## Europäisches Patentamt

## European Patent Office

## Office européen des brevets

(11) Veröffentlichungsnummer: **0 411 166 A1**

# (12) EUROPÄISCHE PATENTANMELDUNG

(21) Anmeldenummer: **89114042.8**

(51) Int. Cl.5: **A61K 7/04**

(22) Anmeldetag: **29.07.89**

(43) Veröffentlichungstag der Anmeldung:
**06.02.91 Patentblatt 91/06**

(84) Benannte Vertragsstaaten:
**AT BE CH DE ES FR GB GR IT LI LU NL SE**

(71) Anmelder: **Dominik, Detlev**
**Augustastrasse 70**
**D-5100 Aachen(DE)**

Anmelder: **HIGH CHEM. SAREMCO S.A./LTD.**
**International Nail Cosmetics Rohnacker**
**CH-9445 Rebstein(CH)**

(72) Erfinder: **Schmid, Adalbert**
**Rohnacker**
**CH-9445 Rebstein(CH)**

(54) **Fingernagel- und Fusspflegeprodukte.**

(57) Als Haftvermittler für Kollagen enthaltende Schichten und Kunststoffe auf Acryl-Basis wird ein Haftvermittler vorgeschlagen, der im wesentlichen aus einem Monomer der Acrylat-Gruppe besteht und aus einem reaktiven Wasserstoff sowie aus einem Stoff, eine Aldehydgruppe enthaltenden Stoff. Die Kombination kann als Mixtur oder als chemisches Reaktionsprodukt ausgebildet sein.

Es wurde festgestellt, daß durch die Verwendung einer derartiger Mixtur im kosmetischen Bereich für Fingernagel- und Fußpflegeprodukte eine optimale Haftung erreicht wird, wenn beispielsweise künstliche Fingernägel und/oder Fußnägel in Verbindung mit dem oben genannten GIUMA-Produkt auf natürliche Nägel aufgeklebt werden. Überraschenderweise ergibt ein Zusatz von Polyvinylpyrrolidone eine weitere Verbesserung der Haftung.

**ZUSATZ ENTWICKLUNG**

Polyvinylpyrrolidone
Plasdone
ergibt eine weitere Verbesserung des Glutaraldehyd / HEMA Haftmittels am Keratin oder Kollagen

| Beispiel 1 | |
|---|---|
| Hydroxyethylmethacrylat | 18,o |
| Urethandimethacr. | 8o,o |
| Lichtinitiatoren | 1,o |
| Glutaraldehyd | 1,o |
| | 1oo,o |

Ergebnis: Gute Haftung am natürlichen Keratin über 4 Wochen.
Kein seitliches Lösen des Kunststoffes feststellbar.

| Beispiel 2 | |
|---|---|
| Hema | 19,5 |
| Glutaraldehyd | 2,o |
| BVP | 5,o |
| BISGMA | 73,o |
| Lichtinitiator | o,5 |
| | 1oo,o |

Excellente Haftung über 4 Wochen ohne sich seitlich vom Keratinzusatz zu lösen.

| Beispiel 3 | |
|---|---|
| Hema | 19,5 |
| Glutaralde hyd | o,1 |
| BVP | 5,o |
| BISGMA | 74,9 |
| Initiator | o,5 |
| | 1oo,o |

Excellente Haftung über 4 Wochen ohne seitliches Ablösen des Kunststoffes vom Keratin.

| Beispiel 4 | |
|---|---|
| Hydroxypropyl-Methacrylat | 2o,o |
| Glutaraldehyd | o,5 |
| BVP | 4,5 |
| BISGMA | 7o,o |
| Ureth.dimetharcylat | 5,o |
| Initiator | o,1 |
| | 1oo,1 |

2

Excellente Haftung über 4 Wochen ohne seitliches Ablösen des Kunststoffes am Keratin.

**Ansprüche**

1. Überraschenderweise wurde gefunden, daß das GIUMA Produkt auch an Finger- und Fussnägeln eine optimale Haftung ergibt.
2. Überraschenderweise ergibt ein Zusatz von PVP (nochmals eine wesentliche Verbesserung in der Haftung am Keratin / Kollagen (PVP = Polyvinylpyrrolidone oder Plasdone)).

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X | EP-A-0 141 324 (BAYER) <br> * Ansprüche; Seite 8, Zeile 4 - Seite 17, Zeile 15; Beispiele 1,2,5,12 * <br> --- | 1 | A 61 K 7/04 |
| X | FR-A-2 022 299 (HYDRON LTD) <br> * Ansprüche; Beispiele 11,12 * <br> --- | 1,2 | |
| X | FR-A-2 240 274 (LEE PHARMACEUTICALS) <br> * Ansprüche; Beispiel; Seite 7, Zeile 39 - Seite 8, Zeile 4 * <br> ----- | 1 | |
| | | | **RECHERCHIERTE SACHGEBIETE (Int. Cl.5)** <br><br> A 61 K |

Der vorliegende Recherchenbericht wurde für alle Patentansprüche erstellt

| Recherchenort | Abschlußdatum der Recherche | Prüfer |
|---|---|---|
| DEN HAAG | 19-06-1990 | WILLEKENS G.E.J. |